# EUROPEAN PATENT APPLICATION

(11) **EP 4 596 074 A1**
(43) Date of publication of application: **06.08.2025**
(21) Application number: 23872391.0
(22) Date of filing: 27.09.2023
(51) Int. Cl.: A63H 11/00

(54) **ROBOT, CONTROL METHOD, AND PROGRAM**

(30) Priority: 29.09.2022 JP 2022156763
(71) Applicant: Nitto Denko Corporation, Ibaraki-shi, Osaka 567-8680 (JP)
(72) Inventor: YAMAUCHI, Kengo, Ibaraki-shi, Osaka 567-8680 (JP); FUKUSHIMA, Rihito, Ibaraki-shi, Osaka 567-8680 (JP); TSURUTA, Hijiri, Ibaraki-shi, Osaka 567-8680 (JP); SHIMIZU, Yusuke, Ibaraki-shi, Osaka 567-8680 (JP)
(74) Representative: Epping - Hermann - Fischer
(86) International application number: PCT/JP2023/035072
(87) International publication number: WO 2024/071169

(57) **Abstract**

A technique in which a robot interacts with a user in a warm state is provided. The robot includes: an acquisition unit configured to acquire information on presence or approach of a user and information on a surface temperature of the robot; and an action control unit configured to instruct to perform an action that temporarily avoids interaction with the user in a case where the surface temperature of the robot does not reach a target temperature when the presence or approach of the user is detected.

## Description

### TECHNICAL FIELD

The present disclosure relates to a robot, a control method, and a program.

### BACKGROUND

Conventionally, a robot designed to interact with a user is known. Additionally, a robot providing comfort by making a user feel the robot's warmth is known.

Patent Document 1 discloses that when an exterior part heated by heat generated inside a robot becomes greater than or equal to a predetermined temperature, the robot reduces heat generation by acting in a way suggesting that a living creature is resting.

### Related Art Document

### Patent Document

[Patent Document 1] Japanese Laid-open Patent Application Publication No. 2009-104878

### SUMMARY OF THE INVENTION

### Problem to be solved by the invention

However, in Patent Document 1, a case where the heating amount of the robot is small is not assumed from the beginning. There is a problem that the power consumption increases when the surface temperature of the robot is constantly controlled. When the temperature control is turned on and off for energy saving, the robot interacts with the user in a state where the robot does not warm up, and cannot provide comfort to the user.

It is an object of the disclosed technique that a robot interacts with a user in a warm state.

### Means for Solving the Problem

An aspect of the present disclosure is a robot including an acquisition unit configured to acquire information on presence or approach of a user and information on a surface temperature of the robot, and an action control unit configured to instruct to perform an action that temporarily avoids interaction with the user in a case the surface temperature of the robot does not reach a target temperature when the presence or approach of the user is detected.

### Effect of the invention

According to an aspect of the present disclosure, a robot can interact with a user in a warm state.

### BRIEF DESCRIPTION OF THE DRAWINGS

[FIG. 1] FIG. 1 is a perspective view of a robot according to an embodiment.
[FIG. 2] FIG. 2 is a side view of the robot according to the embodiment.
[FIG. 3] FIG. 3 is a cross-sectional view of the robot of the embodiment along a cutting line III-III.
[FIG. 4] FIG. 4 is a diagram illustrating a configuration of a vital sensor according to the embodiment.
[FIG. 5] FIG. 5 is a block diagram illustrating a hardware configuration of a controller according to the embodiment.
[FIG. 6] FIG. 6 is a block diagram illustrating a functional configuration of the controller according to the embodiment.
[FIG. 7] FIG. 7 is a flowchart illustrating a process of a control unit according to the embodiment.

### DESCRIPTION OF THE EMBODIMENTS

In the following, embodiments of the present disclosure will be described in detail with reference to the drawings. In the drawings, the same components are denoted by the same reference symbols, and duplicated description will be appropriately omitted.

The embodiments described below are examples of a robot for embodying the technical idea of the present disclosure, and the present disclosure is not limited to the embodiments described below. The dimensions, materials, shapes, relative arrangements, and the like of the components described below are intended to illustrate the examples, not to limit the scope of the present disclosure thereto, unless otherwise specified. Additionally, the size, positional relationship, and the like of members illustrated in the drawings may be exaggerated for clarity of description.

### <Overall Configuration Example of Robot 100>

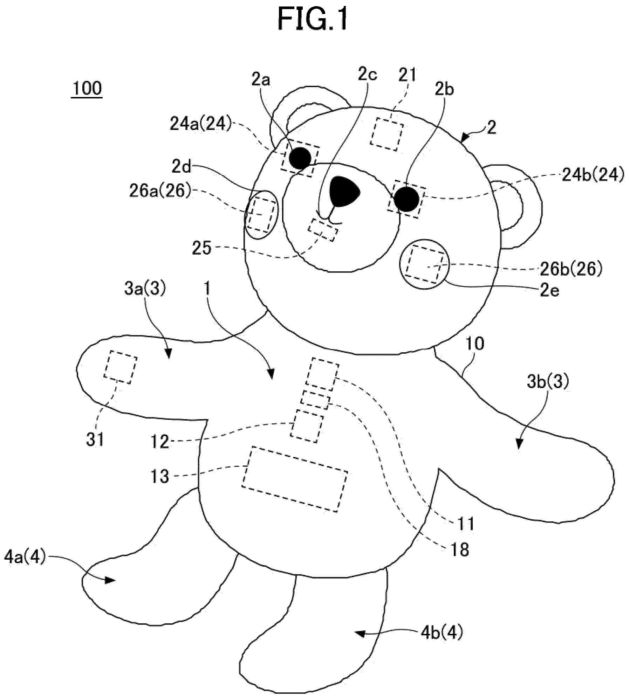

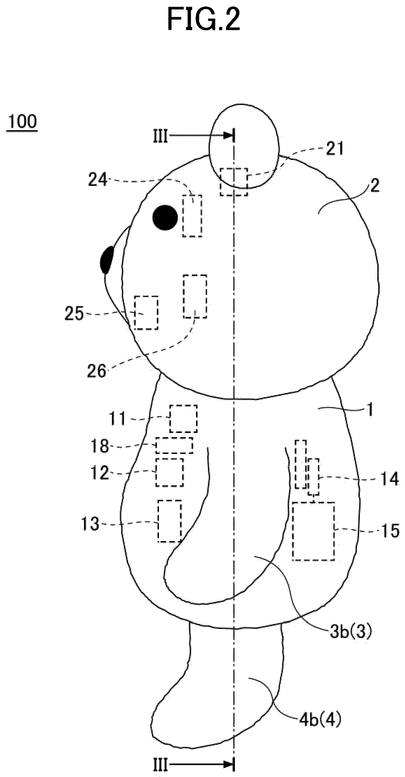

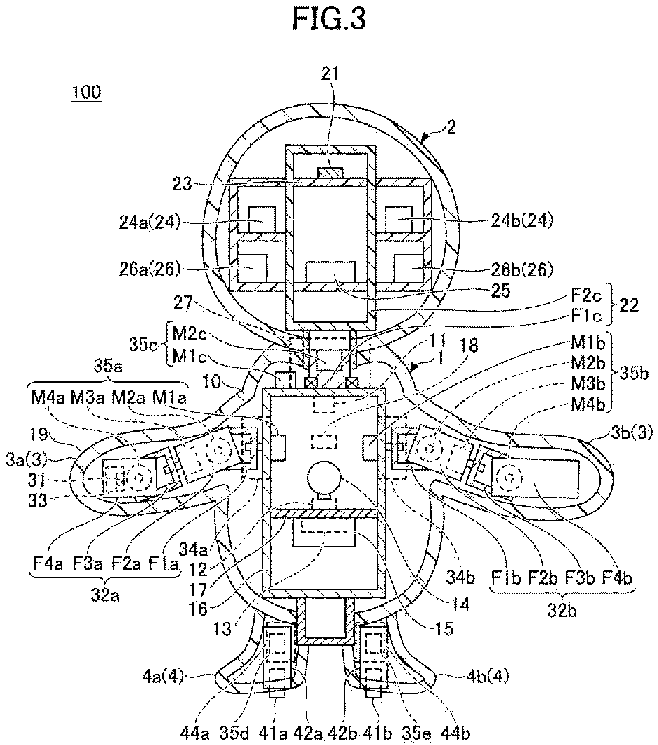

A configuration of a robot 100 according to an embodiment will be described with reference to FIGS. 1 to 3. FIG. 1 is a perspective view illustrating the robot 100 according to the embodiment. FIG. 2 is a side view of the robot 100. FIG. 3 is a cross-sectional view along a cutting line III-III in FIG. 2.

The robot 100 is a robot that includes exterior members 10 and that is configured to be driven by supplied power. The robot 100 described in the present embodiment as an example is a doll-type communication robot in the shape of a bear cub. The robot 100 is manufactured to have a size and weight suitable for being held by a user. Here, the user indicates a user of the robot 100. Typical examples of the user include a working adult living alone, a senior person whose child has moved out, and a frail elderly person who is a target of home healthcare. Additionally, the user may include a contact person who simply contacts the robot 100, such as an administrator of the robot 100, in addition to the user of the robot 100.

The exterior member 10 has flexibility. The exterior member 10 contains, for example, a soft material that is comfortable to touch when the user of the robot 100 touches the robot 100. As the material of the exterior member 10, a material containing an organic material, such as urethane foam, rubber, resin, or fiber can be used. The exterior member 10 preferably includes an exterior, such as a urethane foam material, having a heat insulating property, and a soft cloth material covering the outer surface of the exterior.

In the present embodiment, the cloth material of the exterior member 10 is configured as a heater 19 having a conductive fiber. The heater 19 is provided to cover the entire surface of the exterior, but may be provided to cover a part of the surface of the exterior. Additionally, in another embodiment, when the exterior member 10 is not configured by a urethane foam material or the like having the heat insulation property, but a member having a heat transfer property, the heater 19 may be provided to cover the entire inner surface or a part of the inner surface of the exterior member 10. For example, the part of the inner surface or the part of the surface of the exterior in which the heater 19 is provided is preferably a part that is likely to be touched by a user (a head, an arm, a trunk, or the like).

Additionally, the heater 19 is preferably configured by a positive temperature coefficient (PTC) heater that can adjust its own temperature regardless of the air temperature (the ambient temperature) around the robot 100. Furthermore, the heater 19 may be configured as a heater utilizing exhaust heat inside the robot 100 by connecting a thermal conductive member for transferring exhaust heat from the battery 15 described later, exhaust heat from various sensors, exhaust heat from the controller 13 described later, and the like.

The robot 100 includes, for example, a trunk 1, a head 2, arms 3, and legs 4. The head 2 has a right eye 2a, a left eye 2b, a mouth 2c, a right cheek 2d, and a left cheek 2e. The arms 3 include a right arm 3a and a left arm 3b, and the legs 4 include a right leg 4a and a left leg 4b. Here, the trunk 1 corresponds to a robot main body. Each of the head 2, the arm 3, and the leg 4 corresponds to a driving body that is connected to the robot main body so as to be relatively displaceable.

In the present embodiment, the arm 3 is configured to be displaceable with respect to the trunk 1. For example, when the robot 100 is held by the user, the robot 100 displaces the right arm 3a and the left arm 3b to contact a neck, a trunk, or the like of the user so as to hold the user. This operation allows the user to feel an affinity for the robot 100, and thus promotes the interaction between the user and the robot 100. Here, the interaction with the user indicates an action of the user and the robot 100 touching each other (a contact action), such as rubbing, tapping (touching), hugging (embracing), and the like.

The trunk 1, the head 2, the arms 3, and the legs 4 are all covered with the exterior members 10. The exterior member at the trunk 1 and the exterior member at the arm 3 are integrated, and the exterior members at the head 2 and the leg 4 are separated from the exterior member at the trunk 1 and the arm 3. However, the embodiments are not limited to these configurations, and for example, only a portion of the robot 100 that is likely to be contacted by the user may be covered by the exterior member 10. Additionally, at least one of the exterior members 10 at the trunk 1, the head 2, the arm 3, and the leg 4 may be separated from the other exterior members. Further, a portion in the head 2, the arm 3, and the leg 4 that does not displace may be configured only by the exterior member 10 without including a component such as a sensor therein.

The robot 100 includes a camera 11, a tactile sensor 12, a controller 13, a vital sensor 14, a battery 15, a first capacitive sensor 21, and a second capacitive sensor 31 inside the exterior members 10. Additionally, the robot 100 includes the camera 11, the tactile sensor 12, the controller 13, the vital sensor 14, and the battery 15 inside the exterior member 10 at the trunk 1. Additionally, the robot 100 includes the first capacitive sensor 21 inside the exterior member 10 at the head 2, and the second capacitive sensor 31 inside the exterior member 10 at the arm 3.

Additionally, the robot 100 includes displays 24, a speaker 25, and lights 26 inside the exterior member 10 at the head 2. Additionally, the robot 100 includes the displays 24 inside the exterior member 10 at the right eye 2a and the left eye 2b. Additionally, the robot 100 includes the speaker 25 inside the exterior member 10 at the mouth 2c, and the lights 26 inside the exterior member 10 at the right cheek 2d and the left cheek 2e. Additionally, the robot 100 includes a temperature sensor 18 between the exterior of the exterior member 10 and the cloth material in the trunk 1, but it is preferable to dispose the temperature sensor 18 preferentially in a part likely to be touched by the user (the head, arm, trunk, or the like).

More specifically, as illustrated in FIG. 3, the robot 100 includes a trunk frame 16 and a trunk mounting base 17 inside the exterior member 10 at the trunk 1. Additionally, the robot 100 includes a head frame 22 and a head mounting base 23 inside the exterior member 10 at the head 2. Further, the robot 100 includes a right arm frame 32a and a right arm mounting base 33 inside the exterior member 10 at the right arm 3a, and a left arm frame 32b inside the exterior member 10 at the left arm 3b. In addition, the robot 100 includes a right leg frame 42a inside the exterior member 10 at the right leg 4a, and a left leg frame 42b inside the exterior member 10 at the left leg 4b.

The trunk frame 16, the head frame 22, the right arm frame 32a, the left arm frame 32b, the right leg frame 42a, and the left leg frame 42b are structures each formed by combining multiple columnar members. The trunk mounting base 17, the head mounting base 23, and the right arm mounting base 33 are plate members having placement surfaces. The trunk mounting base 17 is fixed to the trunk frame 16, the head mounting base 23 is fixed to the head frame 22, and the right arm mounting base 33 is fixed to the right arm frame 32a. Here, the trunk frame 16, the head frame 22, the right arm frame 32a, the left arm frame 32b, the right leg frame 42a, and the left leg frame 42b may be formed in a box shape including multiple plate members.

The right arm frame 32a is connected to the trunk frame 16 via a right arm connection mechanism 34a, and is driven by a right arm servo motor 35a, thereby being relatively displaceable with respect to the trunk frame 16. The displacement of the right arm frame 32a causes the right arm 3a to displace relative to the trunk 1. The right arm connection mechanism 34a preferably includes a reduction gear that increases the output torque of the right arm servo motor 35a, for example.

In the present embodiment, the right arm frame 32a is configured by a multi-joint robot arm including multiple frame members and multiple connection mechanisms. For example, the right arm frame 32a includes a right shoulder frame F1a, a right upper arm frame F2a, a right elbow frame F3a, and a right forearm frame F4a. The trunk frame 16, the right shoulder frame F1a, the right upper arm frame F2a, the right elbow frame F3a, and the right forearm frame F4a are connected to each other via respective connection mechanisms.

The right arm servo motor 35a is a generic term of multiple servo motors. For example, the right arm servo motor 35a includes a right shoulder servo motor M1a, a right upper arm servo motor M2a, a right elbow servo motor M3a, and a right forearm servo motor M4a. The right shoulder servo motor M1a rotates the right shoulder frame F1a about a rotation axis perpendicular to the trunk frame 16. The right upper arm servo motor M2a rotates the right upper arm frame F2a about a rotation axis perpendicular to a rotation axis of the right shoulder frame F1a. The right elbow servo motor M3a rotates the right elbow frame F3a about a rotation axis perpendicular to a rotation axis of the right upper arm frame F2a. The right forearm servo motor M4a rotates the right forearm frame F4a about a rotation axis perpendicular to a rotation axis of the right elbow frame F3a.

The left arm frame 32b is connected to the trunk frame 16 via a left arm connection mechanism 34b, and is driven by a left arm servo motor 35b, thereby being relatively displaceable with respect to the trunk frame 16. The displacement of the left arm frame 32b causes the left arm 3b to displace relative to the trunk 1. The left arm connection mechanism 34b preferably includes a reduction gear that increases the output torque of the left arm servo motor 35b, for example.

In the present embodiment, the left arm frame 32b is configured by a multi-joint robot arm including multiple frame members and multiple connection mechanisms. For example, the left arm frame 32b includes a left shoulder frame F1b, a left upper arm frame F2b, a left elbow frame F3b, and a left forearm frame F4b. The trunk frame 16, the left shoulder frame F1b, the left upper arm frame F2b, the left elbow frame F3b, and the left forearm frame F4b are connected to each other via respective connection mechanisms.

The left arm servo motor 35b is a generic term of multiple servo motors. For example, the left arm servo motor 35b includes a left shoulder servo motor M1b, a left upper arm servo motor M2b, a left elbow servo motor M3b, and a left forearm servo motor M4b. The left shoulder servo motor M1b rotates the left shoulder frame F1b about a rotation axis perpendicular to the trunk frame 16. The left upper arm servo motor M2b rotates the left upper arm frame F2b about a rotation axis perpendicular to a rotation axis of the left shoulder frame F1b. The left elbow servo motor M3b rotates the left elbow frame F3b about a rotation axis perpendicular to a rotation axis of the left upper arm frame F2b. The left forearm servo motor M4b rotates the left forearm frame F4b about a rotation axis perpendicular to a rotation axis of the left elbow frame F3b.

The arm 3 includes the four-axis joints as described above, thereby enabling the robot 100 to realize a more realistic operation. For example, when the robot 100 has not warmed up to a target temperature for providing warmth, the robot 100 can act to temporarily avoid interaction with the user by moving the arm 3 and "flapping a hand".

The head frame 22 is connected to the trunk frame 16 via a head connection mechanism 27, and is driven by a head servo motor 35c, thereby being relatively displaceable with respect to the trunk frame 16. The displacement of the head frame 22 causes the head 2 to displace relative to the trunk 1. The head connection mechanism 27 preferably includes a reduction gear that increases the output torque of the head servo motor 35c, for example.

In the present embodiment, the head frame 22 includes a neck frame F1c and a face frame F2c. The trunk frame 16, the neck frame F1c, and the face frame F2c are connected to each other via respective connection mechanisms.

The head servo motor 35c is a generic term of multiple servo motors. For example, the head servo motor 35c includes a neck servo motor M1c and a face servo motor M2c. The neck servo motor M1c rotates the neck frame F1c about a rotation axis perpendicular to the trunk frame 16. The face servo motor M2c rotates the face frame F2c around a rotation axis perpendicular to a rotation axis of the neck frame F1c.

The head 2 includes the two-axis joints as described above, thereby enabling the robot 100 to realize a more realistic operation. For example, when the robot 100 has not warmed up to the target temperature for providing warmth, the robot 100 moves the head 2 and the arm 3 to "dance", so that the robot 100 can act to make the user pay attention to the dancing motion and temporarily avoid interaction with the user.

The right leg frame 42a is connected to the trunk frame 16 via a right leg connection mechanism 44a, and includes a right leg wheel 41a on the bottom side. In order to stabilize the posture of the robot 100, the robot 100 preferably includes two right leg wheels 41a in the front-rear direction of the right leg frame 42a. The right leg wheel 41a is driven by a right leg servo motor 35d, thereby being rotatable about a rotation axis perpendicular to the front-rear direction of the right leg frame 42a. The rotation of the right leg wheel 41a enables the robot 100 to travel. The right leg connection mechanism 44a preferably includes a reduction gear that increases the output torque of the right leg servo motor 35d, for example.

The left leg frame 42b is connected to the trunk frame 16 via a left leg connection mechanism 44b, and includes a left leg wheel 41b on the bottom side. In order to stabilize the posture of the robot 100, the robot 100 preferably includes two left leg wheels 41b in the front-rear direction of the left leg frame 42b. The left leg wheel 41b is driven by the left leg servo motor 35e, thereby being rotatable about a rotation axis perpendicular to the front-rear direction of the left leg frame 42b. The rotation of the left leg wheel 41b enables the robot 100 to travel. The left leg connection mechanism 44b preferably includes a reduction gear that increases the output torque of a left leg servo motor 35e, for example.

In the present embodiment, the robot 100 moves forward or backward by simultaneously rotating the right leg wheel 41a and the left leg wheel 41b forward or backward. The robot 100 turns right or left by braking one of the right leg wheel 41a or the left leg wheel 41b with a brake and turning the other forward or backward.

As described, the robot 100 can realize a more realistic operation by the leg 4. For example, when the robot 100 has not warmed up to the target temperature for providing warmth, the robot 100 can act to temporarily avoid interaction with the user by moving the leg 4 and "step back".

The camera 11 is fixed to the trunk frame 16. The tactile sensor 12, the controller 13, the vital sensor 14, and the battery 15 are fixed to the trunk mounting base 17. The controller 13 and the battery 15 are fixed to the side of the trunk mounting base 17 opposite to the side to which the tactile sensor 12 and the vital sensor 14 are fixed. Here, the arrangement of the controller 13 and the battery 15 is due to the space available on the trunk mounting base 17 and is not limited to the above described arrangement. However, if the battery 15 is fixed to the side of the trunk mounting base 17 opposite to the side to which the tactile sensor 12 and the vital sensor 14 are fixed, the center of gravity of the robot 100 is lowered because the battery 15 is heavier than other components. The center of gravity of the robot 100 is preferably low because at least one of the position or the posture of the robot 100 is stabilized and at least one of charging or replacement of the battery 15 is easily performed.

The first capacitive sensor 21 is fixed to the head mounting base 23, and the second capacitive sensor 31 is fixed to the right arm mounting base 33. The temperature sensor 18 is fixed in connection to or in contact with the heater 19 depending on the sensing method. Alternatively, the temperature sensor 18 may be fixed apart from the heater 19. The display 24 includes a right-eye display 24a and a left-eye display 24b. The right-eye display 24a, the left-eye display 24b, and the speaker 25 are fixed to the head frame 22. The lights 26 include a right-cheek light 26a and a left-cheek light 26b. The right-cheek light 26a and the left-cheek light 26b are fixed to the head frame 22.

Here, the camera 11, the tactile sensor 12, the controller 13, the vital sensor 14, the battery 15, the first capacitive sensor 21, the second capacitive sensor 31, and the like can be fixed by a screw member, an adhesive member, or the like. Additionally, the temperature sensor 18, the right-eye display 24a, the left-eye display 24b, the speaker 25, the right-cheek light 26a, the left-cheek light 26b, and the like can also be fixed by screw members, adhesive members, or the like.

The materials of the trunk frame 16, the trunk mounting base 17, the head frame 22, the head mounting base 23, the right arm frame 32a, the right arm mounting base 33, and the left arm frame 32b are not particularly limited, and a resin material, a metallic material, or the like can be used. However, from the viewpoint of ensuring strength during driving, it is preferable to use a metallic material, such as aluminum, for the trunk frame 16, the right arm frame 32a, and the left arm frame 32b. If the strength can be obtained, it is preferable to use a resin material for the material of each of these parts in order to reduce the weight of the robot 100. The materials of the trunk mounting base 17, the head frame 22, the head mounting base 23, the right arm mounting base 33, and the left arm frame 32b are not particularly limited, and a resin material or a metallic material can be used. However, from the viewpoint of reducing the weight of the robot 100, it is preferable to use a resin material.

The controller 13 is communicably connected to each of the camera 11, the tactile sensor 12, the vital sensor 14, the first capacitive sensor 21, the second capacitive sensor 31, the right arm servo motor 35a, and the left arm servo motor 35b by wire or wirelessly. Additionally, the controller 13 is communicably connected to each of the heater 19, the temperature sensor 18, the head servo motor 35c, the right leg servo motor 35d, and the left leg servo motor 35e by wire or wirelessly. Further, the controller 13 is communicably connected to each of the right-eye display 24a, the left-eye display 24b, the speaker 25, the right-cheek light 26a, and the left-cheek light 26b by wire or wirelessly.

The camera 11 is an image sensor configured to output a captured image of the surroundings of the robot 100 to the controller 13. In the present embodiment, the camera 11 is an example of an imaging section configured to image a user. The camera 11 includes a lens and an imaging element that captures an image formed by the lens. As the imaging element, a charge coupled device (CCD), a complementary metal-oxide semiconductor (CMOS), or the like can be used. The captured image may be either a still image or a moving image.

Additionally, the camera 11 is preferably configured by a time-of-flight (TOF) camera configured to output a distance image of the surroundings of the robot 100 to the controller 13. Therefore, the captured image output from the camera 11 may include a three-dimensional captured image (the distance image) in addition to the two-dimensional captured image or instead of the two-dimensional captured image. The captured image is used for detection of the presence or approach of the user, detection of the distance from the robot 100 to the user, authentication of the user, estimation of emotion or an action of the user, or the like. The captured image is an example of information on the presence or approach of the user. Additionally, the robot 100 may include, in addition to the camera 11 or instead of the camera 11, a human presence sensor, such as an ultrasonic sensor, an infrared sensor, a millimeter wave radar, or a light detection and raging (LiDAR). Sensor information acquired by the human presence sensor is also an example of the information on the presence or approach of the user.

The tactile sensor 12 is a sensor element configured to detect information felt by a tactile sense inherent in a human hand or the like, convert the information into a tactile signal, which is an electrical signal, and output the tactile signal to the controller 13. For example, the tactile sensor 12 converts information on pressure or vibration generated by the user contacting the robot 100 into a tactile signal by a piezoelectric element, and outputs the tactile signal to the controller 13. The tactile signal output from the tactile sensor 12 is used to detect whether a user 200 has contacted the robot 100 or is present.

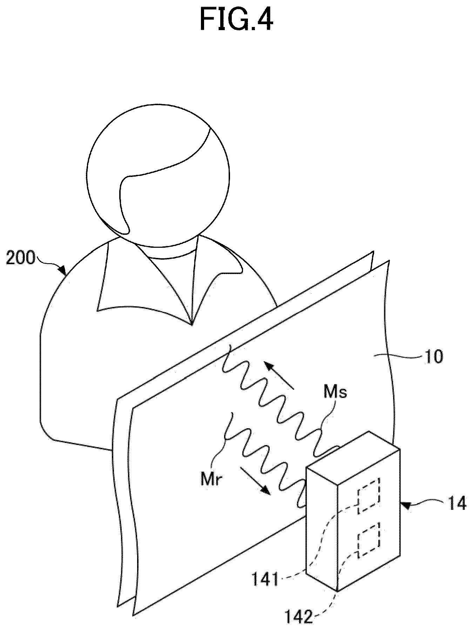

The vital sensor 14 is an example of an electromagnetic wave sensor configured to acquire biological information of the user by using electromagnetic waves. The vital sensor 14 will be described in detail later with reference to FIG. 4.

The first capacitive sensor 21 and the second capacitive sensor 31 are sensor elements configured to output, to the controller 13, a capacitance signal obtained by detecting, based on a change in capacitance, that the user has come into contact with or come in proximity to the robot 100. The first capacitive sensor 21 is preferably a rigid sensor having no flexibility in terms of stabilization of the exterior member 10. The arm 3 is a part that is easily touched by the user, and thus the second capacitive sensor 31 is preferably a sensor having flexibility including a conductive thread or the like from the viewpoint of improving the touch feeling. The capacitance signals output from the first capacitive sensor 21 and the second capacitive sensor 31 are used to detect that the user has come in proximity to the robot 100 or is present.

The temperature sensor 18 is a sensor element configured to output, to the controller 13, a temperature detection signal related to the surface temperature of the robot 100. In the present embodiment, the temperature sensor 18 is configured as an electric circuit configured to output, to the controller 13, a temperature detection signal corresponding to a change in an electrical resistance value of the PTC heater (a change in the current flowing through the heater 19). Additionally, in another embodiment, the temperature sensor 18 may be configured as a contact type temperature sensor (for example, a thermistor, a thermocouple, a platinum thermometer, or the like) that is fixed in contact with the heater 19 and outputs the temperature detection signal to the controller 13. Alternatively, the temperature sensor 18 may be configured by a non-contact type temperature sensor (for example, a radiation temperature sensor) that is fixed apart from the heater 19 and outputs the temperature detection signal to the controller 13. The temperature detection signal output from the temperature sensor 18 is an example of information on the surface temperature of the robot 100. The temperature detection signal is used to detect the surface temperature of the robot 100.

The right-eye display 24a and the left-eye display 24b are display modules configured to display character strings, such as characters, numerals, and symbols, or images in response to a command from the controller 13. The right-eye display 24a and the left-eye display 24b are configured by, for example, liquid crystal display modules. The character strings or images displayed on the right-eye display 24a and the left-eye display 24b are used to express the emotion of the robot 100. For example, when the robot 100 has not warmed up to the target temperature for providing warmth, the robot can act to temporarily avoid the interaction with the user by displaying an image, such as a "thermometer" or an "hourglass", on the right-eye display 24a or the left-eye display 24b.

The speaker 25 is a speaker unit configured to amplify an audio signal from the controller 13 and emit sound. The sound emitted from the speaker 25 is a word or a call of the robot 100, and is used to express the emotion of the robot 100. For example, when the robot 100 has not warmed up to the target temperature for providing warmth, the robot 100 can act to temporarily avoid the interaction with the user by outputting a voice, such as "Wait a minute" or "I am still cold", from the speaker 25.

The right-cheek light 26a and the left-cheek light 26b are light modules configured to blink or change colors in response to an on/off signal from the controller 13. The right-cheek light 26a and the left-cheek light 26b are configured by, for example, light emitting diodes (LEDs). The blinking or the changing of the colors of the right-cheek light 26a and the left-cheek light 26b is used to express the emotion of the robot 100. For example, when the robot 100 has not warmed up to the target temperature for providing warmth, the robot 100 can act to temporarily avoid the interaction with the user by blinking the right-cheek light 26a and the left-cheek light 26b in blue to implicitly indicate that the robot is cold.

The battery 15 is a power supply configured to supply power to each of the camera 11, the tactile sensor 12, the controller 13, the vital sensor 14, the first capacitive sensor 21, the second capacitive sensor 31, the right arm servo motor 35a, and the left arm servo motor 35b. Additionally, the battery 15 supplies power to each of the heater 19, the temperature sensor 18, the head servo motor 35c, the right leg servo motor 35d, and the left leg servo motor 35e. Further, the battery 15 supplies power to each of the right-eye display 24a, the left-eye display 24b, the speaker 25, the right-cheek light 26a, and the left-cheek light 26b. Various secondary batteries, such as a lithium ion battery and a lithium polymer battery can be used as the battery 15.

Here, the various sensors, such as the tactile sensor 12, the vital sensor 14, the first capacitive sensor 21, and the second capacitive sensor 31 in the robot 100 are not essential components. The robot 100 only needs to include at least the camera 11 (or the human presence sensor) and the temperature sensor 18. The installation positions of these components can also be changed as appropriate. Further, the various sensors, such as the camera 11 (or the human presence sensor) and the temperature sensor 18 may be disposed outside the robot 100 and transmit necessary information to the robot 100 or an external device via wireless connection. For example, a learning device configured by a personal computer (PC) or a server is an example of the external device.

Additionally, the robot 100 does not necessarily include the controller 13 inside the exterior member 10, and the controller 13 can communicate with each device from the outside of the exterior member 10 via wireless connection. The battery 15 can supply power to each component from the outside of the exterior member 10.

In the present embodiment, a configuration in which the head 2, the arm 3, and the leg 4 are displaceable is described as an example, but the present embodiment is not limited thereto, and at least one of the head 2, the arm 3, or the leg 4 may be displaceable. Additionally, the arm 3 is configured by a four-axis multi-joint robot arm, but may be configured by a six-axis multi-joint robot arm. Further, the arm 3 is preferably connectable to an end effector, such as a hand. Additionally, the leg 4 is configured by a wheel system, but can be configured by a crawler system, a leg system, or the like.

The configuration and shape of the robot 100 are not limited to those examples described in the present embodiment, and can be appropriately changed according to the preference of the user, the use form of the robot 100, and the like. For example, the robot 100 may be in a form of a robot arm, such as an industrial robot, or a form of a human, such as a humanoid, instead of a form of imitating a bear cub. Additionally, the robot 100 may be in a form of a mobile device, such as a drone or a vehicle, including at least one of an arm, a display, a speaker, a light, and the like.

### <Configuration Example of Vital Sensor 14>

FIG. 4 is a diagram illustrating a configuration of the vital sensor 14. The vital sensor 14 is a microwave Doppler sensor including a microwave transmitter 141 and a microwave receiver 142. The microwave is an example of the electromagnetic wave.

The vital sensor 14 transmits a transmitted wave Ms, which is a microwave, toward the user 200 from the inside of the exterior member 10 of the robot 100 by the microwave transmitter 141. The vital sensor 14 receives, by the microwave receiver 142, a reflected wave Mr of the transmitted wave Ms that is reflected by the user 200.

The vital sensor 14 detects, in a non-contact manner, a minute displacement generated on a body surface due to the beating of the heart of the user 200 or the like, based on a difference between the frequency of the transmitted wave Ms and the frequency of the reflected wave Mr, by using the Doppler effect. The vital sensor 14 can acquire information, such as a heartbeat, respiration, a pulse wave, and a blood pressure, as the biological information of the user 200 based on the detected minute displacement, and output the acquired biological information to the controller 13.

However, the vital sensor 14 is not limited to the microwave Doppler sensor, and may be a sensor configured to detect a minute displacement generated on a body surface by using a change in coupling between a human body and an antenna, or may be a sensor configured to use an electromagnetic wave other than the microwave, such as near-infrared light. Additionally, the vital sensor 14 may be a millimeter wave radar, a microwave radar, or the like. Further, the vital sensor 14 preferably includes a non-contact thermometer configured to detect infrared rays or the like transmitted from the user 200 in addition to the Doppler sensor. In this case, the vital sensor 14 detects biological information of the user 200 including information on at least one of a heartbeat (a pulse), respiration, a blood pressure, or a body temperature.

In the present embodiment, the vital sensor 14 is provided inside the exterior member 10, and thus the user 200 cannot visually recognize the vital sensor 14. This suppresses the user 200 from feeling resistance to the detection of the biological information, and enables the biological information to be smoothly acquired. Additionally, the vital sensor 14 can acquire the biological information in a non-contact manner, and thus can acquire the biological information even when the user moves to some extent, unlike a contact sensor that requires the user to be in contact with the same place for a certain period of time.

Additionally, by promoting the interaction between the user 200 and the robot 100 by the hug operation of the robot 100 or the like, the robot 100 is held by the user 200 and can acquire the biological information in a state of being in contact with or in proximity to the user 200. With this, the robot 100 can acquire highly reliable biological information in which noise is suppressed.

### <Configuration Example of Controller 13>

### (Hardware Configuration Example)

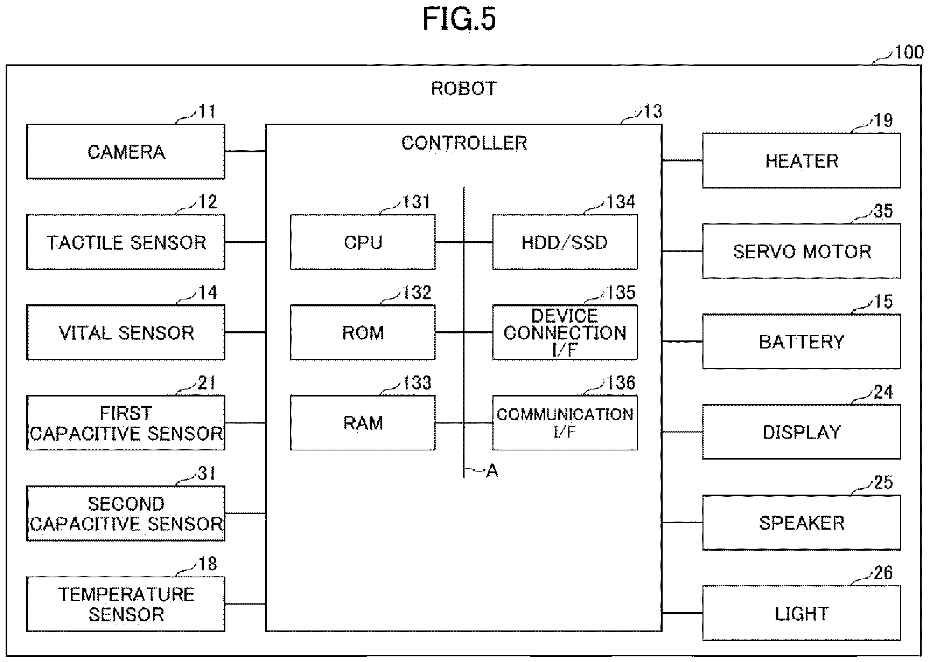

FIG. 5 is a block diagram illustrating a hardware configuration of the controller 13. The controller 13 is constructed by a computer, and includes a central processing unit (CPU) 131, a read only memory (ROM) 132, and a random access memory (RAM) 133. Additionally, the controller 13 includes a hard disk drive/solid state drive (HDD/SSD) 134, a device connection interface (I/F) 135, and a communication I/F 136. These are communicably connected to each other via a system bus A.

The CPU 131 executes control processing including various arithmetic processing. The ROM 132 stores a program used to drive the CPU 131, such as an initial program loader (IPL). The RAM 133 is used as a work area of the CPU 131. The HDD/SSD 134 stores various information, such as programs, captured images acquired by the camera 11, detection information by various sensors, such as the biological information acquired by the vital sensor 14, the temperature detection signal acquired by the temperature sensor 18, and the like.

The device connection I/F 135 is an interface for connecting the controller 13 to various external devices. The external devices herein are the camera 11, the tactile sensor 12, the vital sensor 14, the first capacitive sensor 21, the second capacitive sensor 31, a servo motor 35, the battery 15, and the like. Additionally, the external devices include the temperature sensor 18, the heater 19, the display 24, the speaker 25, the light 26, and the like.

Here, the servo motor 35 is a generic term of the right arm servo motor 35a, the left arm servo motor 35b, the head servo motor 35c, the right leg servo motor 35d, and the left leg servo motor 35e. Additionally, the display 24 is a generic term for the right-eye display 24a and the left-eye display 24b. Further, the light 26 is a generic term for the right-cheek light 26a and the left-cheek light 26b.

The communication I/F 136 is an interface for communicating with an external device via a communication network or the like. For example, the controller 13 is connected to the Internet via the communication I/F 136 and communicates with the external device via the Internet.

Here, at least some of the functions realized by the CPU 131 may be realized by an electric circuit or an electronic circuit.

### (Functional Configuration Example)

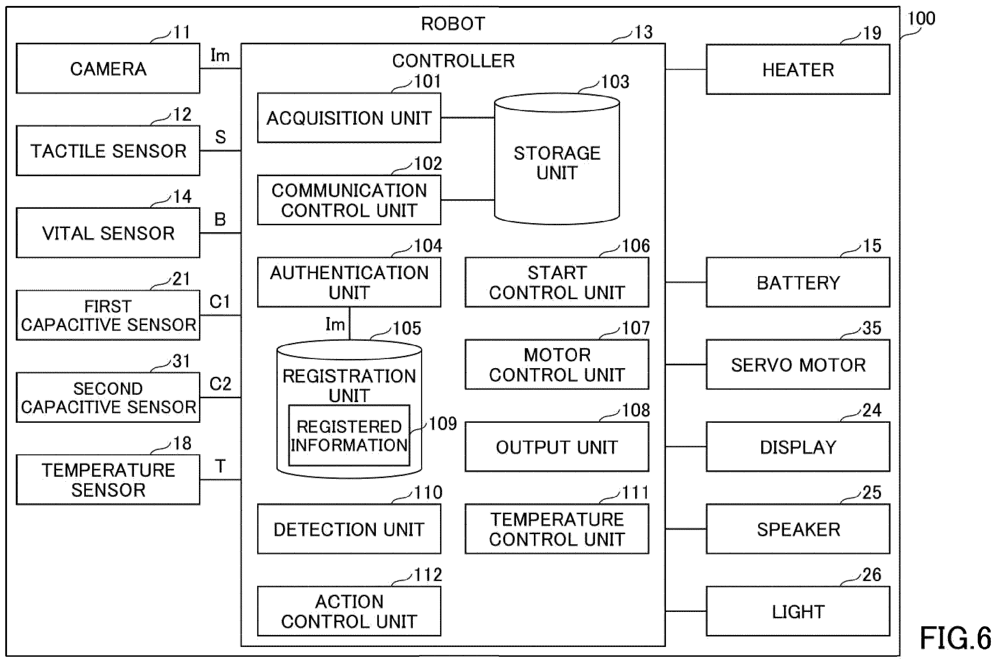

FIG. 6 is a block diagram illustrating a functional configuration of the controller 13. The controller 13 includes an acquisition unit 101, a communication control unit 102, a storage unit 103, an authentication unit 104, a registration unit 105, a start control unit 106, a motor control unit 107, an output unit 108, a detection unit 110, a temperature control unit 111, and an action control unit 112.

The controller 13 can realize the functions of the acquisition unit 101 and the output unit 108 by the device connection I/F 135 or the like, and can realize the function of the communication control unit 102 by the communication I/F 136 or the like. Additionally, the controller 13 can realize the functions of the storage unit 103 and the registration unit 105 by a non-volatile memory, such as the HDD/SSD 134. Further, the functions of the authentication unit 104, the start control unit 106, and the motor control unit 107 can be realized by a processor, such as the CPU 131, executing processing defined in a program stored in a non-volatile memory, such as the ROM 132.

Additionally, the functions of the detection unit 110, the temperature control unit 111, and the action control unit 112 can be realized by a processor, such as the CPU 131, executing processing defined in a program stored in a non-volatile memory, such as the ROM 132. Here, some of the above-described functions of the controller 13 may be realized by an external device, such as a PC or a server, or may be realized by distributed processing between the controller 13 and the external device.

The acquisition unit 101 controls communication between the controller 13 and the camera 11 to acquire a captured image Im from the camera 11. Additionally, the acquisition unit 101 controls communication between the controller 13 and the tactile sensor 12 to acquire a tactile signal S from the tactile sensor 12. Further, the acquisition unit 101 controls communication between the controller 13 and the vital sensor 14 to acquire biological information B from the vital sensor 14.

Additionally, the acquisition unit 101 controls communication between the controller 13 and the first capacitive sensor 21 to acquire a first capacitance signal C1 from the first capacitive sensor 21. Additionally, the acquisition unit 101 controls communication between the controller 13 and the second capacitive sensor 31 to acquire a second capacitance signal C2 from the second capacitive sensor 31. Furthermore, the acquisition unit 101 acquires a temperature detection signal T from the temperature sensor 18 by controlling communication between the controller 13 and the temperature sensor 18.

The communication control unit 102 controls communication with an external device via a communication network or the like. For example, the communication control unit 102 can transmit the captured image Im acquired by the camera 11, the biological information B acquired by the vital sensor 14, the tactile signal S acquired by the tactile sensor 12, and the like to the external device (for example, a learning device) via the communication network.

The storage unit 103 stores the biological information B acquired by the vital sensor 14. The storage unit 103 continuously stores the acquired biological information B while the acquisition unit 101 is acquiring the biological information B from the vital sensor 14. Additionally, the storage unit 103 can continuously store information obtained from the captured image Im of the camera 11, the tactile signal S of the tactile sensor 12, the first capacitance signal C1 of the first capacitive sensor 21, the second capacitance signal C2 of the second capacitive sensor 31, and the temperature detection signal T of the temperature sensor 18.

The authentication unit 104 performs personal authentication of the user 200 based on the captured image Im of the user 200 captured by the camera 11. For example, the authentication unit 104 performs face authentication by referring to registered information 109 of a face image registered in advance in the registration unit 105, based on the captured image Im including the face of the user 200 captured by the camera 11. With this, the user 200 currently in contact with or in proximity to the robot 100 can be associated with the personal information registered in advance, and the biological information B acquired by the vital sensor 14 can be associated with the personal information. Additionally, the controller 13 can also perform control so as to stop the start of the acquisition of the biological information by the vital sensor 14 when the face image included in the captured image Im is not registered in the registration unit 105.

The start control unit 106 causes the vital sensor 14 to start acquiring the biological information B. For example, when the contact or proximity of the user 200 with respect to the robot 100 is detected by the detection unit 110, the start control unit 106 turns on a switch or the like for supplying power from the battery 15 to the vital sensor 14. With this, the start control unit 106 causes the vital sensor 14 to start acquiring the biological information B.

The detection unit 110 detects the presence or approach of the user 200 around the robot 100 based on the captured image Im and the like obtained by the camera 11. The detection unit 110 preferably detects the distance from the robot 100 to the user 200 based on the captured image Im (the distance image) obtained by the camera 11. Additionally, the detection unit 110 may detect the presence or proximity of the user 200 with respect to the robot 100 based on the first capacitance signal C1 or the second capacitance signal C2. Further, the detection unit 110 detects the presence or contact of the user 200 with respect to the robot 100 based on the tactile signal S from the tactile sensor 12.

The temperature control unit 111 controls the surface temperature of the robot 100. The temperature control unit 111 detects the surface temperature of the robot 100 based on the temperature detection signal T when the presence or approach of the user 200 is detected by the detection unit 110. The temperature detection signal T may not directly represent the surface temperature of the robot 100 due to the arrangement, the configuration, and the like of the heater 19 or the temperature sensor 18. Therefore, the temperature control unit 111 estimates the surface temperature of the robot 100 from the temperature detection signal T.

The temperature control unit 111 adjusts the temperature of the heater 19 so that the surface temperature of the robot 100 reaches the target temperature. The temperature control unit 111 adjusts the temperature of the entire surface or a part of the surface of the robot 100. If the temperature of the heater 19 can be adjusted for each part of the robot 100 (for example, each of the trunk 1, the head 2, the arm 3, and the leg 4), the temperature control unit 111 may preferentially adjust the temperature of a part of the surface of the robot 100 (for example, the head 2) that is likely to be touched by the user 200.

Additionally, when the heater 19 is configured by the PTC heater that can adjust the temperature by itself, the temperature control unit 111 only needs to turn on the switch of the PTC heater while the presence or approach of the user 200 is detected. When the heater 19 is configured by a general heater other than the PTC heater, the temperature control unit 111 adjusts the temperature of the heater 19 so that a difference between the surface temperature of the robot 100 and the target temperature approaches zero while the presence or approach of the user 200 is detected. As the temperature control method, it is preferable to use proportional control (P control), proportional integral control (PI control), proportional integral derivative control (PID control), or the like.

Additionally, during the temperature adjustment, the temperature control unit 111 may calculate the time to be taken for the surface temperature of the robot 100 to reach the target temperature, based on a change rate of the surface temperature of the robot 100 (for example, [°C/s]) and store it in the storage unit 103. Additionally, the function of calculating the time required for reaching the target temperature may be performed by an external device.

The storage unit 103 stores the target temperature of the robot 100. In order for the user 200 to feel warmth, the target temperature of the robot 100 is preferably set to be a temperature (for example, 37°C to 40°C) slightly higher than the temperature of human skin (for example, 35°C to 38°C). Additionally, because the surface temperature of the robot 100 changes in accordance with the ambient temperature around the robot 100, the target temperature of the robot 100 is preferably changed in accordance with the ambient temperature. Furthermore, because the temperature at which the user 200 feels warmth is different for each user 200, the set value of the target temperature of the robot 100 is preferably configured such that the user 200 can change the set value.

In a case where the surface temperature of the robot 100 does not reach the target temperature when the presence or approach of the user 200 is detected by the detection unit 110, the action control unit 112 instructs to perform the action that temporarily avoids the interaction with the user 200. With this, the time until the surface temperature of the robot 100 reaches the target temperature can be gained. By interacting with the user 200 while the robot 100 is in a warm state, comfort can be provided to the user 200.

Here, the action that temporarily avoids the interaction with the user 200 does not need be an action in which the robot 100 dislikes interaction with the user 200. The action that temporarily avoids the interaction with the user 200 is an action to gain the time until the surface temperature of the robot 100 reaches the target temperature at which the user 200 feels warmth, before the interaction with the user 200. Therefore, the action that temporarily avoids the interaction with the user 200 is an action that prepares for the interaction with the user 200 while inducing the interaction with the user 200.

For example, the action that avoids the interaction with the user 200 preferably includes an action that makes the user 200 pay attention to the action of the robot 100, such as "dance" or "pose". Additionally, the action that avoids the interaction with the user 200 may include an action that draws the attention of the user 200 to a place different from the robot 100, such as "point with a finger". Additionally, the "action that draws the attention of the user 200" includes an action to guide the user's attention to an object other than the robot 100 by stimulating the five senses of the user 200, and may be, for example, an action such as "output a voice of 'Look over there'". Additionally, the action that avoids the interaction with the user 200 preferably includes an action that implicitly indicates that the user is preparing, such as "rub hands" or "warm up". Further, the action that avoids the interaction with the user 200 may include an action to clearly notify the user of the avoidance of the interaction with the user 200, such as "display a thermometer" or output a voice of "Wait a minute".

The storage unit 103 stores information on a predefined action an (n is an identification number of the action) of the robot 100. The information on the action an of the robot 100 is managed by, for example, a table of a database. The following table 1 is an example of an action table TB1 related to the action an of the robot 100. The action table TB1 includes an action ID for identifying the action an of the robot 100, an action content of the robot 100, a command content of the action an, the action time per one cycle, and an example of use.

**[Table 1]**

| ACTION ID | ACTION CONTENT | COMMAND CONTENT | ACTION TIME | EXAMPLE OF USE |
|---|---|---|---|---|
| a0 | DANCE (DANCE BY MOVING AT LEAST ONE OF ARM, LEG, OR HEAD) | 35: TAUGHT COMMAND, ROTATIONAL SPEED 200 mm/s | 15 s/cycle | DIVERT ATTENTION TO ACTION TO AVOID INTERACTION |
| a1 | FLAP HANDS (SPREAD HANDS AND FLAP HANDS UP AND DOWN) | 35: TAUGHT COMMAND, ROTATIONAL SPEED 200 mm/s | 3 s/cycle | IMPLICITLY AVOID INTERACTION |
| a2 | EMIT SOUND (EMIT SOUND, SUCH AS "JUST A MINUTE" OR "I AM STILL COLD") | 25: EMIT SOUND | 2 s/cycle | EXPLICITLY AVOID INTERACTION |
| a3 | CHANGE EXPRESSION 1 (CAUSE TO FLICKER/ CHEEK COLOR TO CHANGE) | 26: FLICKER/ CHANGE COLOR | 5 s/cycle | IMPLICITLY AVOID INTERACTION |
| a4 | CHANGE EXPRESSION 2 (DISPLAY THERMOMETER OR HOURGLASS) | 24: DISPLAY IMAGE | 3 s/cycle | EXPLICITLY AVOID INTERACTION |
| a5 | CHANGE EXPRESSION 3 (OPEN AND CLOSE EYES) | 24: DISPLAY IMAGE (MAY BE COMMAND TO PHYSICALLY MOVE EYELID) | 2 s/cycle | IMPLICITLY AVOID INTERACTION |
| a6 | CHANGE EXPRESSION 4 (CHANGE PUPIL OR IRIS SIZE AND CAUSE EYES TO GLOW RED) | 24: DISPLAY IMAGE | 2 s/cycle | IMPLICITLY AVOID INTERACTION |
| a7 | CHANGE EXPRESSION 5 (DISPLAY OR MOVE NOSE, MOUTH, EYEBROW, AND EARS) | 24: DISPLAY IMAGE | 3 s/cycle | IMPLICITLY AVOID INTERACTION |
| a8 | CHANGE EXPRESSION 6 (MOVE EYES TO FOLLOW PERSON) | 24: DISPLAY IMAGE | 10 s/cycle | IMPLICITLY AVOID INTERACTION |
| a9 | CHANGE EXPRESSION 7 (DISPLAY TEXT OR SYMBOLS DIRECTLY IN EYES) | 24: DISPLAY CHARACTER STRING | 3 s/cycle | EXPLICITLY AVOID INTERACTION |
| a10 | MOVE AROUND IN PLACE (SPIN AROUND TO MAKE IT DIFFICULT TO BE CAPTURED) | 35: TAUGHT COMMAND, ROTATIONAL SPEED 200 mm/s | 8 s/cycle | EXPLICITLY AVOID INTERACTION |
| a11 | STEP BACK (BACK AWAY) | 35: TAUGHT COMMAND, ROTATIONAL SPEED 100 mm/s | 2 s/cycle | EXPLICITLY AVOID INTERACTION |
| a12 | ROLL AWAY (ROLL AWAY ON FLOOR TO MAKE IT DIFFICULT TO BE CAPTURED) | 35: TAUGHT COMMAND, ROTATIONAL SPEED 100 mm/s | 5 s/cycle | IMPLICITLY AVOID INTERACTION |
| a13 | SHIVER (SHIVER AND LOOK COLD) | 35: TAUGHT COMMAND, ROTATIONAL SPEED 200 mm/s | 5 s/cycle | IMPLICITLY AVOID INTERACTION |
| a14 | RUB HANDS TOGETHER (RUB HANDS TOGETHER TO TRY TO WARM HANDS UP) | 35: TAUGHT COMMAND, ROTATIONAL SPEED 200 mm/s | 3 s/cycle | IMPLICITLY AVOID INTERACTION (DEMONSTRATE BEING IN PREPARATION) |
| a15 | WARM UP (WARM UP BODY BY STRETCHING, HIGH KNEES, OR THE LIKE) | 35: TAUGHT COMMAND, ROTATIONAL SPEED 200 mm/s | 8 s/cycle | IMPLICITLY AVOID INTERACTION (DEMONSTRATE BEING IN PREPARATION) |
| a16 | POSE (PERFORMING SERIES OF POSING MOVEMENTS) | 35: TAUGHT COMMAND, ROTATIONAL SPEED 200 mm/s | 10 s/cycle | DIVERT ATTENTION TO ACTION TO AVOID INTERACTION |
| a17 | POINT WITH FINGER (DIRECT YOUR ARM OR HAND TOWARD OBJECT OR PERSON) | 35: TRACKING COMMAND + TAUGHT COMMAND, ROTATIONAL SPEED 200 mm/s | 3 s/cycle | ATTRACT ATTENTION TO DIFFERENT PLACE TO AVOID INTERACTION |
| an | ... | ... | ... | ... |

A symbol in the command content in Table 1 is a symbol representing a control target. Additionally, a taught command is an operation command that is previously taught using a teaching method, such as offline teaching, online teaching, or direct teaching. Additionally, a tracking command is an operation command to track the position and posture of the user 200 based on various sensor information, such as the captured image Im (the distance image).

Preferably, the action control unit 112 determines the action an that avoids the interaction with the user 200 based on the distance from the robot 100 to the user 200 and instructs to perform the action an. For example, when the distance from the robot 100 to the user 200 is relatively long (for example, three meters or greater), the action control unit 112 instructs to perform the action an to make the user 200 pay attention to the action of the robot 100, such as "dance" or "pose". Alternatively, the action control unit 112 may instruct to perform an action an having a long action time per cycle, such as "move around in place" or "warm up".

Additionally, when the distance from the robot 100 to the user 200 is relatively medium (for example, one meter or greater and less than three meters), the action control unit 112 instructs to perform the action an to indicate that the robot 100 is preparing, such as "rub hands" or "warm up". Alternatively, the action control unit 112 may instruct to perform the action an that draws the attention to a place different from the robot 100, such as "point with a finger".

Furthermore, when the distance from the robot 100 to the user 200 is relatively short (for example, less than one meter), the action control unit 112 may instruct to perform the action an that explicitly avoids the interaction with the user 200, such as outputting a voice, such as "Wait a minute", or displaying a "thermometer". Alternatively, the action control unit 112 may instruct to perform the action an having a short action time per cycle, such as "step back" or output a voice that says "I am still cold".

Additionally, the action control unit 112 may determine the action an that avoids the interaction with the user 200 based on the time to be taken for the surface temperature of the robot 100 to reach the target temperature and instruct to perform the action an. When the time to be taken for the surface temperature of the robot 100 to reach the target temperature is relatively long (for example, greater than three minutes), the action control unit 112 may instruct to perform the action an that makes the user 200 pay attention to the action of the robot 100, such as "dance" or "pose". Alternatively, the action control unit 112 may instruct to perform the action an having a long action time per cycle, such as "move around in place" or "warm up".

Additionally, when the time to be taken for the surface temperature of the robot 100 to reach the target temperature is relatively medium (for example, one minute or greater and less than three minutes), the action control unit 112 may instruct to perform the action an that indicates that the robot 100 is preparing, such as "rub hands" or "warm up". Alternatively, the action control unit 112 may instruct to perform the action an that draws the attention to a place different from the robot 100, such as "point with a finger".

Furthermore, when the time to be taken for the surface temperature of the robot 100 to reach the target temperature is relatively short (for example, less than one minute), the action control unit 112 instructs to perform the action an that explicitly avoids the interaction with the user 200, such as outputting a voice, such as "Wait a minute". Alternatively, the action control unit 112 may instruct to perform the action an having a short action time per cycle, such as "step back" or output a voice that says "I am still cold".

With the description above, the time to be taken for the surface temperature of the robot 100 to reach the target temperature is gained. Here, the action that temporarily avoids the interaction with the user 200 may be any combination of the multiple types of actions an illustrated in Table 1. For example, the action control unit 112 may instruct to perform multiple types of actions an in a sequence of "dance" -> "warm up" -> "wait a minute".

Additionally, in another embodiment, the action control unit 112 may instruct to perform the action at (t: time) that temporarily avoids the interaction with the user 200 in accordance with the state st (t: time) of the user 200 or randomly.

For example, the state st of the user 200 is a predetermined combination of the emotion and action of the user 200 estimated based on the captured image Im of the user 200 and the biological information B of the user 200. The estimation of the emotion or action of the user 200 is preferably performed by performing machine learning or by using a learned learning model. As the learning method, reinforcement learning, supervised learning, unsupervised learning, semi-supervised learning, or the like can be used. Additionally, as the learning model, an action value table, a neural network, or the like can be used.

For example, if the presence or approach of the user 200 in a state of running in irritation is detected, the user 200 is highly likely not to interact with the robot 100. The action at of displaying, for example, "I am still cold" to such a user 200 gives the user 200 unnaturalness in communication.

Therefore, it is preferable that the action control unit 112 determines the action at suitable for the state st of the user 200 by performing machine learning or using the learned learning model, and instructs to perform the action at. The action control unit 112 generates and updates, using machine learning, a learning model configured to receive the state st of the user 200 and output the value of the action at. Alternatively, the action control unit 112 may randomly determine the action at so that the user 200 does not get bored with the patterned action of the robot 100, and instruct to perform the action at. Here, the learning process and the determination process of the action at suitable for the state st of the user 200 may be performed by an external device communicably connected to the robot 100.

The motor control unit 107 controls the drive of the servo motor 35 in response to the action control unit 112 instructing to perform the action an of the robot 100. When the action content of the robot 100 is, for example, "dance", the motor control unit 107 performs a previously taught operation command, such as "dance" or "warm up".

The output unit 108 controls communication between the controller 13 and the display 24 in response to the action control unit 112 instructing to perform the action at of the robot 100. When the action content of the robot 100 is, for example, displaying "I am still cold," the output unit 108 outputs the character string data of "I am still cold" to the right-eye display 24a and the left-eye display 24b. When the action content of the robot 100 is, for example, displaying a "thermometer", the output unit 108 outputs the image data of the "thermometer" to the right-eye display 24a and the left-eye display 24b.

Additionally, the output unit 108 controls communication between the controller 13 and the speaker 25 in response to the action control unit 112 instructing to perform the action at of the robot 100. When the action content of the robot 100 is, for example, outputting a voice that says "Wait a minute", the output unit 108 outputs a voice output signal of "Wait a minute" to the speaker 25.

Furthermore, the output unit 108 controls communication between the controller 13 and the light 26 in response to the action control unit 112 instructing to perform the action at of the robot 100. When the action content of the robot 100 is, for example, "blinking the cheek", the output unit 108 turns on and off the switching elements of the right-cheek light 26a and the left-cheek light 26b at predetermined time intervals.

### <Example of Process by Controller 13>

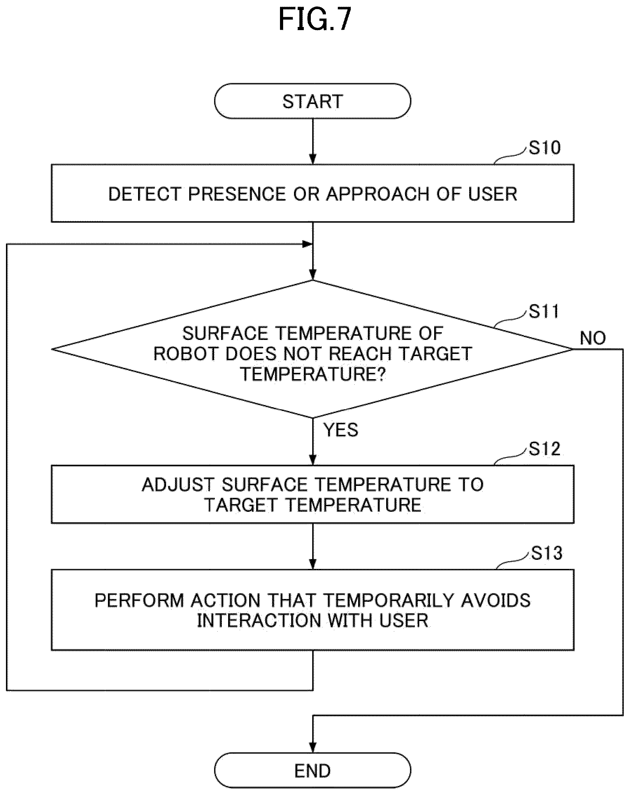

FIG. 7 is a flowchart illustrating a process of the controller 13. FIG. 7 illustrates the process in which the controller 13 executes an operation command to temporarily avoid the interaction with the user 200 when the surface temperature of the robot 100 does not reach the target temperature.

First, in step S10, the controller 13 detects the presence or approach of the user 200 around the robot 100 by the detection unit 110 based on various sensor information, such as the captured image Im. Additionally, if the captured image Im is a distance image, the controller 13 preferably detects the distance from the robot 100 to the user 200 by the detection unit 110.

Here, in step S10, power is supplied to the camera 11, the tactile sensor 12, the first capacitive sensor 21, and the second capacitive sensor 31 from the battery 15. However, in order to reduce the power consumption of the battery 15, power is not supplied to the heater 19, the vital sensor 14, the temperature sensor 18, the servo motor 35, the display 24, the speaker 25, and the light 26.

Next, in step S11, the controller 13 detects the surface temperature of the robot 100 by the temperature control unit 111 based on the temperature detection signal T, and determines whether the surface temperature of the robot 100 reaches the target temperature. Here, the target temperature of the robot 100 is read from the storage unit 103 by the temperature control unit 111.

If the surface temperature of the robot 100 does not reach the target temperature (YES in step S11), in step S12, the controller 13 adjusts the surface temperature of the robot 100 to the target temperature by the temperature control unit 111. The controller 13 adjusts the temperature of the entire surface of the robot 100, but may preferentially adjust the temperature of a part of the surface of the robot 100 that is likely to be touched by the user 200 (for example, the head 2).

Additionally, if the heater 19 is configured by the PTC heater that can adjust the temperature by itself, the temperature control unit 111 only needs to turn on the switch of the PTC heater while the presence or approach of the user 200 is detected. If the heater 19 is configured by a general heater other than the PTC heater, the temperature control unit 111 turns on the switch of the heater 19 to adjust the temperature while the presence or approach of the user 200 is detected.

Then, in step S13, the controller 13 instructs, by the action control unit 112, to perform the action an of the robot 100 temporarily avoiding the interaction with the user 200. The controller 13 may determine the action an of the robot 100 temporarily avoiding the interaction with the user 200 based on the distance from the robot 100 to the user 200 by the action control unit 112 and instruct to perform the action an.

For example, when the distance from the robot 100 to the user 200 is relatively long (for example, three meters or greater), the controller 13 instructs, by the action control unit 112, to perform the action an that makes the user 200 pay attention to the action of the robot 100, such as "dance" or "pose". Alternatively, the controller 13 may instruct, by the action control unit 112, to perform the action an having a long action time per cycle, such as "move around in place" or "warm up".

Additionally, when the distance from the robot 100 to the user 200 is relatively medium (for example, one meter to three meters), the controller 13 instructs, by the action control unit 112, to perform the action an indicating that the robot 100 is preparing, such as "rub your hands" or "warm up". Alternatively, the action control unit 112 may instruct to perform the action an that draws the attention to a place different from the robot 100, such as "point with a finger".

Furthermore, when the distance from the robot 100 to the user 200 is relatively short (for example, less than one meter), the controller 13 instructs, by the action control unit 112, to perform the action an that explicitly avoids the interaction with the user 200, such as output a voice that says "Wait a minute". Alternatively, the controller 13 may instruct, by the action control unit 112, to perform the action an having a short action time per cycle, such as "step back" or output a voice that says "I am still cold".

Additionally, in step S13, the controller 13 may instruct, by the action control unit 112, to determine the action an that avoids the interaction with the user 200 based on the time to be taken for the surface temperature of the robot 100 to reach the target temperature, and perform the action an.

When the time to be taken for the surface temperature of the robot 100 to reach the target temperature is relatively long (for example, three minutes or greater), the controller 13 instructs, by the action control unit 112, to perform the action an that makes the user 200 pay attention to the action of the robot 100, such as "dance" or "pose". Alternatively, the controller 13 may instruct, by the action control unit 112, to perform the action an having a long action time per cycle, such as "move around in place" or "warm up".

When the time to be taken for the surface temperature of the robot 100 to reach the target temperature is relatively medium (for example, one minute to three minutes), the controller 13 instructs, by the action control unit 112, to perform the action an that indicates that the robot 100 is preparing, such as "warm up". Alternatively, the controller 13 may instruct, by the action control unit 112, to perform the action an that draws the attention to a place different from the robot 100, such as "point with a finger".

Furthermore, when the time to be taken for the surface temperature of the robot 100 to reach the target temperature is relatively short (for example, less than one minute), the controller 13 instructs, by the action control unit 112, to perform the action an that explicitly avoids the interaction with the user 200, such as display a "thermometer". Alternatively, the controller 13 may instruct, by the action control unit 112, to perform the action an having a short action time per cycle, such as "step back" or output a voice that says "I am still cold".

As described, the time to be taken for the surface temperature of the robot 100 to reach the target temperature is gained. Here, the action that temporarily avoids the interaction with the user 200 may be a combination of multiple actions an indicated in Table 1.

Additionally, in another embodiment, the controller 13 may instruct, by the action control unit 112, to perform the action at (t: time) that temporarily avoids the interaction with the user 200 in accordance with the state st (t: time) of the user 200 or randomly. For example, the controller 13 instructs, by the action control unit 112, to determine the action at suitable for the state st of the user 200 by performing machine learning or using a learned learning model, and perform the action at. Alternatively, the controller 13 instructs, by the action control unit 112, to randomly determine the action at and perform the action at so that the user 200 does not get bored with the patterned action of the robot 100.

While the robot 100 performs the action that temporarily avoids the interaction with the user 200 in step S13, the controller 13 determines, by the temperature control unit 111, whether the surface temperature of the robot 100 has reached the target temperature in step S11. If the surface temperature of the robot 100 has reached the target temperature (NO in step S11), the controller 13 ends the temperature control by the temperature control unit 111 and ends the action an, by the action control unit 112, that temporarily avoids the interaction.

As described above, in the case where the surface temperature of the robot 100 does not reach the target temperature when the presence or approach of the user is detected, the controller 13 performs a process of instructing to perform the action an that temporarily avoids the interaction with the user 200.

Here, at the start of the process illustrated in FIG. 7, the heater 19, the vital sensor 14, the temperature sensor 18, the servo motor 35, the display 24, the speaker 25, and the light 26 may be in a standby state (a sleep state) in which the amount of supplied power is suppressed. That is, the controller 13 may suppress the power consumption of the battery 15 by returning various devices as necessary from the standby state in which the amount of supplied power is suppressed.

### <Operation Effect of Present Embodiment>

As described above, the robot 100 includes the action control unit 112 configured to instruct to perform the action an that temporarily avoids the interaction with the user 200 in the case where the surface temperature of the robot 100 does not reach the target temperature when the presence or approach of the user 200 is detected.

By the robot 100 performing the action an that temporarily avoids the interaction with the user 200 and gaining the time until the robot 100 warms up, the robot 100 can interact with the user 200 in a warm state. Additionally, the robot 100 does not need to constantly control the warmth, and thus it has the technical advantages of reducing the power consumption of the battery 15, reducing the number of charges, and reducing the battery weight.

Additionally, the action an of the robot 100 that temporarily avoids the interaction with the user 200 includes an action that makes the user 200 pay attention to the action of the robot 100, such as "dance", or the action that draws the attention of the user 200 to a place different from the robot 100, such as "point with a finger". Therefore, the user 200 does not feel stress of waiting, and rather, is highly likely to feel a positive impression, such as cute or funny, of the robot 100.

Additionally, the robot 100 can determine the action an that temporarily avoids the interaction with the user 200 based on the distance from the robot 100 to the user 200. For example, when the distance to the user 200 is relatively long, by performing the action that makes the user 200 pay attention to the action of the robot 100, the time until the robot warms up can be gained while inducing the interaction with the user 200. When the distance to the user 200 is relatively short, by performing the action that clearly notifies the avoidance of the interaction with the user 200, the robot 100 can prevent the interaction with the user 200 in a non-warm state.

Furthermore, the robot 100 can determine the action an that avoids the interaction with the user 200 based on the time to be taken for the surface temperature of the robot 100 to reach the target temperature. For example, when the time to be taken for the surface temperature of the robot 100 to reach the target temperature is relatively long, by performing the action that makes the user 200 pay attention to the action of the robot 100, the time until the robot warms up can be gained while inducing the interaction with the user 200. When the time to be taken for the surface temperature of the robot 100 to reach the target temperature is relatively short, by performing the action that clearly notifies the avoidance of the interaction with the user 200, the robot 100 can prevent the interaction with the user 200 in a non-warm state.

Additionally, the robot 100 can preferentially adjust the temperature of a part of the surface of the robot 100 (for example, the head 2, or the like) that is likely to be touched by the user 200. With this, the robot 100 can prevent the interaction with the user 200 in a non-warm state.

Although the preferred embodiments have been described in detail above, the present invention is not limited to the above-described embodiments, and various modifications and substitutions can be made to the above-described embodiments without departing from the scope of the appended claims.

Additionally, the numerals, such as ordinal numbers and quantities used in the description of the above-described embodiments are all examples for specifically describing the technique of the present invention, and the present invention is not limited to the numerals described as examples. Additionally, the connection relationship between the constituent elements is an example for specifically describing the technique of the present invention, and the connection relationship for realizing the functions of the present invention is not limited thereto.

The robot 100 according to the present embodiment is particularly suitable for use in promoting oxytocin secretion and providing comfort (sense of security or self-affirmation) to a working adult living alone, a senior person whose child has moved out, a frail elderly person who is a target of home healthcare, or the like. However, the robot 100 is not limited to this use, and can be used to provide comfort to various users.

Aspects of the present disclosure are as follows, for example.
<1> A robot including:
   an acquisition unit configured to acquire information on presence or approach of a user and information on a surface temperature of the robot; and
   an action control unit configured to instruct to perform an action that temporarily avoids interaction with the user in a case where the surface temperature of the robot does not reach a target temperature when the presence or approach of the user is detected.
<2> The robot as described in <1>, wherein the action that temporarily avoids the interaction with the user includes an action that makes the user pay attention to an action of the robot or includes an action that draws attention of the user to a place different from the robot.
<3> The robot as described in <1> or <2>, wherein the action that temporarily avoids the interaction with the user includes an action that implicitly indicates that the robot is preparing or includes an action that explicitly indicates avoidance of the interaction.
<4> The robot as described in any one of <1> to <3>, wherein the action control unit determines the action that temporarily avoids the interaction with the user based on a distance from the robot to the user and instructs to perform the action.
<5> The robot as described in any one of <1> to <4>, wherein the action control unit determines the action that temporarily avoids the interaction with the user based on a time until the surface temperature of the robot reaches the target temperature and instructs to perform the action.
<6> The robot as described in any one of <1> to <5>, wherein the action control unit instructs to perform the action that temporarily avoids the interaction with the user in accordance with a state of the user or randomly.
<7> The robot as described in any one of <1> to <6>, further including a temperature control unit configured to preferentially adjust a temperature of a part of a surface of the robot.
<8> The robot as described in any one of <1> to <7>, further including a camera or a human presence sensor configured to acquire a captured image used to detect the presence or approach of the user.
<9> The robot as described in any one of <1> to <8>, further including:
   an exterior member configured as a PTC heater; and
   a temperature sensor configured to output a temperature detection signal in accordance with a change in an electric resistance value of the PTC heater.
<10> A control method of a robot, the control method including:
   a step of acquiring, by the robot, information on presence or approach of a user and information on a surface temperature of the robot; and
   a step of performing, by the robot, an action that temporarily avoids interaction with the user in a case where the surface temperature of the robot does not reach a target temperature when the presence or approach of the user is detected.
<11> A program for causing a computer controlling a robot to execute:
   a step of acquiring information on presence or approach of a user and information on a surface temperature of the robot; and
   a step of instructing to perform an action that temporarily avoids interaction with the user in a case where the surface temperature of the robot does not reach a target temperature when the presence or approach of the user is detected.
<12> A robot including an action control unit configured to instruct to perform, based on information on presence or approach of a user and information on a surface temperature of the robot, an action that temporarily avoids interaction with the user.

This application is based on and claims priority to Japanese Patent Application No. 2022-156763 filed in the Japan Patent Office on September 29, 2022, the entire contents of which are incorporated herein by reference.

### Description of reference symbols

1 trunk
2 head
2a right eye
2b left eye
2c mouth
2d right cheek
2e left cheek
3 arm
3a right arm
3b left arm
4 leg
4a right leg
4b left leg
10 exterior member
11 camera
12 tactile sensor
13 controller
14 vital sensor (electromagnetic wave sensor)
141 microwave transmitter
142 microwave receiver
15 battery
16 trunk frame
17 trunk mounting basel
18 temperature sensor
19 heater
21 first capacitive sensor
22 head frame
23 head mounting base
24 display
24a right-eye display
24b left-eye display
25 speaker
26 light
26a right-cheek light
26b left-cheek light
27 head connection mechanism
31 second capacitive sensor
32a right arm frame
32b left arm frame
33 right arm mounting base
34a right arm connection mechanism
34b left arm connection mechanism
35 servo motor
35a right arm servo motor
35b left arm servo motor
35c head servo motor
35d right leg servo motor
35e left leg servo motor
41a right leg wheel
41b left leg wheel
42a right leg frame
42b left leg frame
44a right leg connection mechanism
44b left leg connection mechanism
100 robot
101 acquisition unit
102 communication control unit
103 storage unit
104 authentication unit
105 registration unit
106 start control unit
107 motor control unit
108 output unit
109 registered information
110 detection unit
111 estimation unit
112 action control unit
131 CPU
132 ROM
133 RAM
134 HDD/SSD
135 device connection I/F
136 communication I/F
200 user
A system bus
B biological information
C1 first capacitance signal
C2 second capacitance signal
F1a right shoulder frame
F2a right upper arm frame
F3a right elbow frame
F4a right forearm frame
F1b left shoulder frame
F2b left upper arm frame
F3b left elbow frame
F4b left forearm frame
F1c neck frame
F2c face frame
Im captured image
L error
LM learning model
Ms transmitted wave
Mr reflected wave
M1a right shoulder servo motor
M2a right upper arm servo motor
M3a right elbow servo motor
M4a right forearm servo motor
M1b left shoulder servo motor
M2b left upper arm servo motor
M3b left elbow servo motor
M4b left forearm servo motor
M1c neck servo motor
M2c face servo motor

## Claims

1. A robot comprising:
an acquisition unit configured to acquire information on presence or approach of a user and information on a surface temperature of the robot; and
an action control unit configured to instruct to perform an action that temporarily avoids interaction with the user in a case where the surface temperature of the robot does not reach a target temperature when the presence or approach of the user is detected.

2. The robot as claimed in claim 1, wherein the action that temporarily avoids the interaction with the user includes an action that makes the user pay attention to an action of the robot or includes an action that draws attention of the user to a place different from the robot.

3. The robot as claimed in claim 1 or 2, wherein the action that temporarily avoids the interaction with the user includes an action that implicitly indicates that the robot is preparing or includes an action that explicitly indicates avoidance of the interaction.

4. The robot as claimed in claim 1 or 2, wherein the action control unit determines the action that temporarily avoids the interaction with the user based on a distance from the robot to the user and instructs to perform the action.

5. The robot as claimed in claim 1 or 2, wherein the action control unit determines the action that temporarily avoids the interaction with the user based on a time until the surface temperature of the robot reaches the target temperature and instructs to perform the action.

6. The robot as claimed in claim 1 or 2, wherein the action control unit instructs to perform the action that temporarily avoids the interaction with the user in accordance with a state of the user or randomly.

7. The robot as claimed in claim 1 or 2, further comprising a temperature control unit configured to preferentially adjust a temperature of a part of a surface of the robot.

8. The robot as claimed in claim 1 or 2, further comprising a camera or a human presence sensor configured to acquire a captured image used to detect the presence or approach of the user.

9. The robot as claimed in claim 1 or 2, further comprising:
an exterior member configured as a PTC heater; and
a temperature sensor configured to output a temperature detection signal in accordance with a change in an electric resistance value of the PTC heater.

10. A control method of a robot, the control method comprising:
a step of acquiring, by the robot, information on presence or approach of a user and information on a surface temperature of the robot; and
a step of performing, by the robot, an action that temporarily avoids interaction with the user in a case where the surface temperature of the robot does not reach a target temperature when the presence or approach of the user is detected.

11. A program for causing a computer controlling a robot to execute:
a step of acquiring information on presence or approach of a user and information on a surface temperature of the robot; and
a step of instructing to perform an action that temporarily avoids interaction with the user in a case where the surface temperature of the robot does not reach a target temperature when the presence or approach of the user is detected.

12. A robot comprising an action control unit configured to instruct to perform, based on information on presence or approach of a user and information on a surface temperature of the robot, an action that temporarily avoids interaction with the user.
